# EUROPEAN PATENT SPECIFICATION

(11) **EP 2 826 503 B1**
(45) Date of publication and mention of the grant of the patent: **03.05.2017**
(21) Application number: 13761915.1
(22) Date of filing: 22.02.2013
(51) Int. Cl.: A61M 1/02, A61M 1/38, B04B 5/04, F16J 15/34, B04B 7/08

(54) **BLOOD COMPONENTS SEPARATION DEVICE, AND CENTRIFUGAL SEPARATOR**
BLUTKOMPONENTENTRENNVORRICHTUNG UND ZENTRIFUGALABSCHEIDER
DISPOSITIF DE SÉPARATION DE COMPOSANTS SANGUINS ET SÉPARATEUR CENTRIFUGE

(30) Priority: 13.03.2012 JP 2012055878
(43) Date of publication of application: 21.01.2015
(73) Proprietor: Terumo Kabushiki Kaisha, Tokyo 151-0072 (JP)
(72) Inventor: IGARASHI Masatsugu, Fujinomiya-shi Shizuoka 418-0004 (JP)
(74) Representative: Casalonga
(86) International application number: PCT/JP2013/054491
(87) International publication number: WO 2013/136943

(56) References cited:
- JP-A- 2001 079 452
- JP-A- 2006 247 217
- JP-A- 2006 247 217
- JP-A- 2010 119 913
- JP-B2- 4 808 219
- JP-B2- H0 545 187
- JP-B2- H0 714 496
- US-A- 3 565 330
- US-A- 4 753 729
- US-A1- 2009 309 308

## Description

### Technical Field

This invention relates to a blood component separation device having a centrifugal separator including a stator provided with an inlet and an outlet of blood or a blood component and a rotor provided rotatably with respect to the stator, and to a centrifugal separator used in the device.

In particular the present invention relates to a blood component separation device according to the preamble of claim 1, such as it is for example known from US 4 753 729A, and a centrifugal separator according to the preamble of claim 2, such as it is for example known from JP 2006 247217 A.

### Background Art

Conventionally, blood collection is perf ormedby two blood collection methods: whole blood collection and blood component collection. The blood component collection is separated to plasma collection and platelet collection. When platelet is necessary for treatment, a method for collecting platelet only and returning the other components to a blood donor is preferable. In order to take out platelet from blood, a blood component separation device provided with a centrifugal separator is used.

A centrifugal separator described in Patent Literature 1 includes a stator provided with an inlet and an outlet of blood or a blood component and a rotor provided rotatably with respect to the stator. The rotor of the centrifugal separator is rotated with respect to the stator inside the blood separation device. At this time, a ring on rotor side and a ring on stator side are in contact with and slide on each other, so that abnormal noise is generated. It is disclosed that the technique of Patent Literature 1 can prevent generation of abnormal noise by providing a damping member on the outer peripheral of the stator and providing an elastic body at a part of attaching the ring on rotor side.

### Citation List

### Patent Literature

Patent Literature 1: JP 2006-247217 A

### Summary of Invention

### Technical Problem

However, the centrifugal separator using the technique of Patent Literature 1 still has the following issue.

In order to centrifuge blood by a centrifugal separator, priming of anticoagulant on the inner surface of tubes or the centrifugal separator where blood passes is necessary before introducing blood therein. Thereafter, blood is introduced into the centrifugal separator for centrifugation. With the technique of Patent Literature 1, abnormal noise can be reduced when blood is in the centrifugal separator, but it has an issue that noise around the high frequency band (high-pitched noise) is generated when the centrifugal separator is rotated in a process of priming anticoagulant. The high-pitched noise is not generated by all centrifugal separators and the incidence of noise generation is low. However, high-pitched noise is confusing with mechanical noise and causes an issue of imparting anxiety to an operator and a blood donor.

The present invention is made in view of the above situations and an object thereof is to provide a blood separation device that does not generate high-pitched noise even when a centrifugal separator is rotated before blood is introduced into the centrifugal separator.

### Solution to Problem

In order to achieve the above-described object, the present invention provides a blood component separation device according to claim 1 and a centrifugal separator according to claim 2.

### Advantageous Effects of Invention

Since the blood component separation device according to the present invention is structured according to claim 1, the blood component separation device provides operation and effect to be described below.

The inventor of the present invention attempted various measures to prevent generation of high-pitched noise by changing roughness of the rotating and sliding portion between the stator and the rotor, for example. However, high-pitched noise generation couldn't be suppressed to none. Finally, the inventor found that high-pitched noise generation could be suppressed by setting the rubber hardness of the ring cap to between 60 and 66 inclusive. The reason will be described below.

Stick-slip phenomenon on the sliding surface generated by the rotor with respect to the stator can be suppressed, and thus high-pitched noise generation can be suppressed.

Conventionally, the rotating and sliding portion has been pressed by high pressing force in order to enhance sealing performance. The inventor of the present invention has found that stick-slip is therefore generated and high-pitched noise is generated upon slipping. The inventor has therefore considered that high-pitched noise generation can be suppressed if stick-slip phenomenon can be suppressed and confirmed the fact by an experiment.

In addition, the inventor of the present invention has found that that pressing force at the rotating and sliding portion between the stator and the rotor can be 500 grams or less by setting the rubber hardness to 66 or less.

More specifically, in the conventional blood component separation device, the rotor and the stator of the centrifugal separator has been pressed for a predetermined distance when the centrifugal separator is fitted. Because of this, the predetermined pressing force becomes 500 grams or more when the rubber hardness of the ring cap is 66 or more and thus elastic deformation of the ring cap is small. Thus, stick-slip is generated. In addition, since the rubber hardness of 60 or less degrades sealing performance, the rubber hardness is set to 60 or more.

Further, high-pitched noise generation can be suppressed.

In addition, the centrifugal separator according to the present invention is structured as described above, and thus provides operation and effect to be described below.

Stick-slip generated by the fixing ring with respect to the resin ring can be suppressed and thus high-pitched noise generation can be suppressed when the rotor is rotated in a state where blood is introduced into the centrifugal separator.

### Brief Description of Drawings

FIG. 1 is a drawing for illustrating a first process (priming process) of a blood component separation device according to the embodiment.
FIG. 2 is a drawing for illustrating a second process of the embodiment.
FIG. 3 is a drawing for illustrating a third process (critical flow process) of the embodiment.
FIG. 4 is a drawing for illustrating a fourth process (circulation flow process) of the embodiment.
FIG. 5 is a drawing for illustrating a process of collecting low concentration platelet solution in a fifth process (acceleration process) of the embodiment.
FIG. 6 is a drawing for illustrating a process of storing high concentration platelet solution in the fifth process (acceleration process) of the embodiment.
FIG. 7 is a drawing illustrating a process of collecting low concentration platelet solution in the fifth process (acceleration process) of the embodiment.
FIG. 8 is a half longitudinal cross-sectional view of a centrifugal separator of the embodiment.
FIG. 9 is a side view of the blood component separation device in a state where a centrifugal bowl of the embodiment is arranged therein but not yet set.
FIG. 10 is a side view of the blood component separation device in a state where the centrifugal bowl of the embodiment is arranged therein and has been set.
FIG. 11 is an enlarged view of a seal mechanism portion of the embodiment illustrated in FIG. 9.
FIG. 12 is an enlarged view of the seal mechanism portion of the embodiment illustrated in FIG. 10.
FIG. 13 is a half longitudinal cross-sectional view of a state where whole blood is supplied in the centrifugal bowl of the embodiment and blood components are separated by centrifugal force.
FIG. 14 is a graph of correlation between pressure and vibration acceleration according to the embodiment.
FIG. 15 is a graph of correlation between the hardness and the pressure of a ring cap of the embodiment.
FIG. 16 is drawing illustrating a configuration of the blood component separation device according to the embodiment.

### Description of Embodiments

First, an embodiment of the present invention will be described with reference to accompanying drawings.

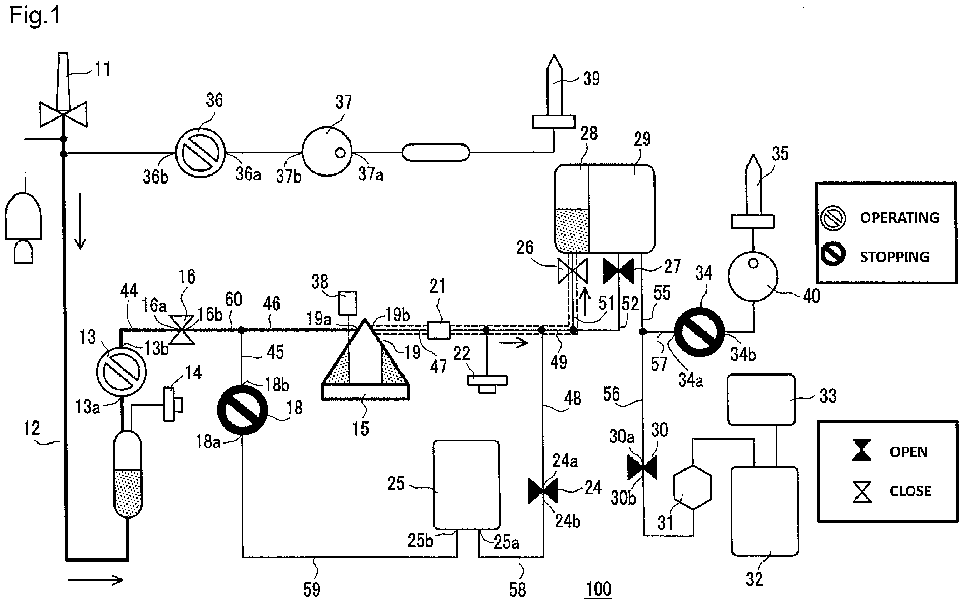

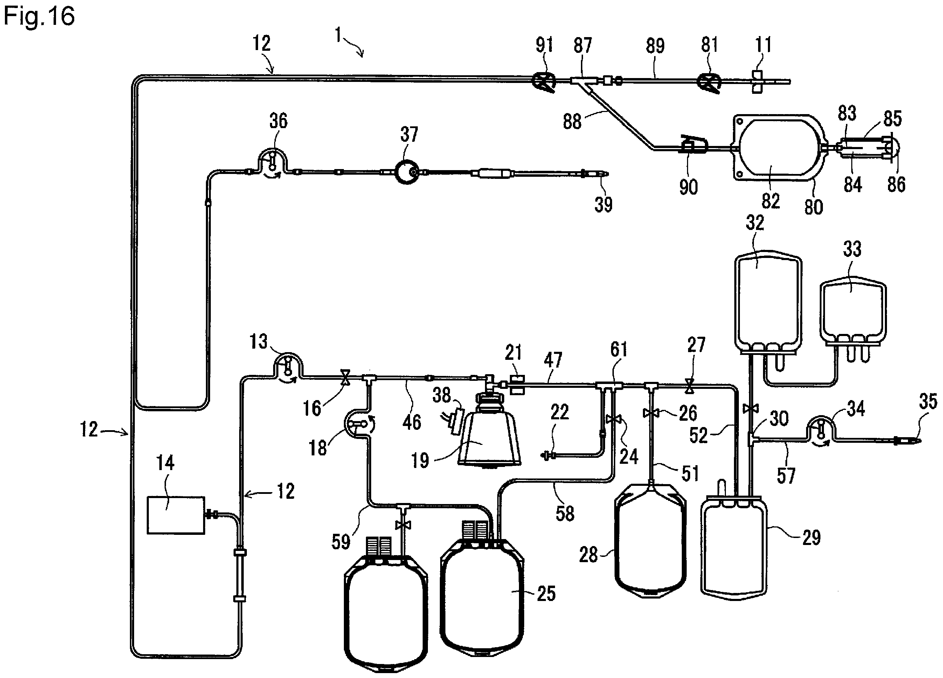

FIG. 1 illustrates a system configuration of a blood component separation device 100 according to the embodiment. FIG. 16 illustrates a configuration of the blood component separation device. The blood component separation device 100 according to the embodiment includes a blood component separation circuit 1 as illustrated in FIG. 16. The blood component separation circuit 1 includes a collection needle 11, an initial flow blood collection circuit 80 for collecting initial flow blood, a rotor having a blood storage space therein , a centrifugal bowl driving device 15 that rotatably drives the rotor, and an inlet 19a and an outlet 19b. The initial flow blood collection circuit 80 includes an initial flow blood collection bag 82, a sampling port 85, and an initial flow blood collection line 88. The blood component separation device 100 also includes a centrifugal bowl 19 that separates blood into a plurality of blood components by rotation of the rotor, a first container (plasma bag) 25, a second container (air bag) 28, and a third container (platelet intermediate bag) 29 that store blood components separated by the centrifugal bowl 19, a first line (a donor tube 12, a first blood pump 13, a tube 44, a first on-off valve 16, a tube 60, and a tube 46) that connects between the collection needle 11 and the centrifugal bowl 19, a second line (a tube 47, a tube 48, a second on-off valve 24, and a tube 58) that connects between the centrifugal bowl 19 and the first container 25, a third line (a tube 59, a second blood pump 18, and a tube 45) that connects between the first container 25 and the first line, a fourth line (the tube 47, a tube 49, a tube 51, and a third on-off valve 26) that connects between the centrifugal bowl 19 and the air bag 28, and a fifth line (the tube 47, the tube 49, a tube 52, and a fourth on-off valve 27) that connects between the centrifugal bowl 19 and the third container 29.

The collection needle 11 for collecting whole blood from a blood donor is connected to one end of the donor tube 12. The other end of the donor tube 12 is connected to a first port 13a of the first blood pump 13. The initial flow blood collection bag 82 is connected to a blood collection needle from a branch part provided on the donor tube 12 by the initial flow blood collection line 88. The initial flow blood collection bag 82 further includes the sampling port 85 for transporting collected initial flow blood to a container for examination, which is not illustrated. The sampling port 85 includes a needle part 83, a body section 86, and a cover part 84 that covers the needle part. In addition, on the initial flow blood collection line 88, a clamp 90 for opening/closing the line is provided. The tube 44 connected to a second port 13b of the first blood pump 13 is connected to a first port 16a of the first on-off valve 16. A pressure sensor 14 is connected to the donor tube 12. The tube 60 connected to a second port 16b of the first on-off valve 16 is branched into two tubes. The tube 45, which is one of the tubes, is connected to an output port 18b of the second blood pump 18. The tube 46, which is the other of the tubes, is connected to the inlet 19a of the centrifugal bowl 19, which is a centrifugal separator. More specifically, the collection needle 11 and the inlet 19a of the centrifugal bowl 19 are connected by the first line (the donor tube 12, the first blood pump 13, the tube 44, the first on-off valve 16, the tube 60, and the tube 46).

The tube 47 connected to the outlet 19b of the centrifugal bowl 19 is branched into two tubes. The tube 48, which is one of the tubes, is connected to an input port 24a of the second on-off valve 24, and the tube 49, which is the other of the two tubes, is further branched to be described later. In the middle of the tube 47 connected to the outlet 19b of the centrifugal bowl 19, a turbidity sensor 21, and a pressure sensor 22 are attached. In addition, in the peripheral part of the part where the centrifugal bowl 19 is attached, an interface sensor 38 for detecting the interface position of a buffy coat layer formed in the centrifugal bowl 19 is attached. An output port 24b of the second on-off valve 24 is connected to an input port 25a of the plasma bag 25 by the tube 58. More specifically, the outlet 19b of the centrifugal bowl 19 and the input port 25a of the plasma bag 25 are connected by the second line (the tube 47, the tube 48, the second on-off valve 24, and the tube 58) .

An output port 25b of the plasma bag 25 is connected to an input port 18a of the second blood pump 18 by the tube 59. The output port 18b of the second blood pump 18 is connected to the tube 45. More specifically, the output port 25b of the plasma bag 25 and the first line are connected by the third line (the tube 59, the second blood pump 18, and the tube 45) . Thus, the plasma bag 25 is connected in a manner such that the plasma bag 25 communicates with the inlet 19a and the outlet 19b of the centrifugal bowl 19. The communication is open/shut off by operation of the second blood pump 18 and the second on-off valve 24.

The tube 49 branched off from the tube 47 is branched into two tubes as described above. The tube 51, which is one of the tubes, is connected to the air bag 28 through the third on-off valve 26. The tube 52, which is the other of the tubes, is connected to the platelet intermediate bag 29 through the fourth on-off valve 27. More specifically, the outlet 19b of the centrifugal bowl 19 and the air bag 28 are connected by the fourth line (the tube 47, the tube 49, the tube 51, and the third on-off valve 26), the outlet 19b of the centrifugal bowl 19 and the platelet intermediate bag 29 are connected by the fifth line (the tube 47, the tube 49, the tube 52, and the fourth on-off valve 27). Thus, the platelet intermediate bag 29 is connected so as to selectively communicate with the outlet 19b of the centrifugal bowl 19 and the communication is open/shut off by operation of, the second on-off valve 24, the third on-off valve 26, and the fourth on-off valve 27.

A tube 55 running out from the platelet intermediate bag 29 is branched into two tubes. A tube 56, which is one of the tubes, is connected to an input port 30a of a fifth on-off valve 30, and a tube 57, which is the other of the tubes, is connected to an output port 34b of a third blood pump 34. An input port 34a of the third blood pump 34 is connected to a platelet storage liquid bottle, which is not illustrated, by a platelet bottle needle 35 through a second sterile filter 40. An output port 30b of the fifth on-off valve 30 is connected to a platelet bag 32 through a leukocyte removing filter 31. To the platelet bag 32, an air bag 33 is connected.

Meanwhile, in the middle of the donor tube 12, an output port 36b of an ACD pump 36 is connected. An input port 36a of the ACD pump 36 is connected to an output port 37b of a first sterile filter 37 . An input port 37a of the first sterile filter 37 is connected to an ACD storage bottle by an ACD bottle needle 39.

To a control unit, which is not illustrated, the first blood pump 13, the second blood pump 18, the third blood pump 34, the centrifugal bowl driving device 15, the ACD pump 36, the turbidity sensor 21, the interface sensor 38, the pressure sensor 22, the first on-off valve 16, the second on-off valve 24, the third on-off valve 26, the fourth on-off valve 27, and the fifth on-off valve 30 are electrically connected. The centrifugal bowl 19 is arranged on the centrifugal bowl driving device 15, which is a rotatably driving unit, and rotatably driven.

Preferable constituent materials of the tubes include polyesters such as polyvinyl chloride, polyethylene, polypropylene, PET, and PBT and various thermoplastic elastomers such as ethylene vinyl acetate copolymer (EVA), polyurethane, and polyester elastomer, for example. Among them, polyvinyl chloride is particularly preferable. Polyvinyl chloride can provide sufficient flexibility and easy handling and is suitable for closing by a clamp or the like.

As a constituent material of the bags, a polymer obtained by polymerizing or copolymerizing soft olefins or diolefins such as polyvinyl chloride, polyolefin, ethylene, propylene, butadiene, and isoprene, in which DEHP is used as a plasticizer may be used. In addition, any combination thereof such as an ethylene vinyl acetate copolymer (EVA), and a polymer blend of EVA and various thermoplastic elastomers may be used. Further, PET, PBT, PCGT, etc. canbeused. Amongthem, polyvinylchlorides are particularly preferable. For containers for storing platelet, a material having excellent gas permeability is preferable in order to improve storability of platelet. Thus, for containers for storing platelet, polyolefin, DnDP-plasticized polyvinyl chloride, etc. may be preferably used or a sheet having a reduced thickness may be preferably used.

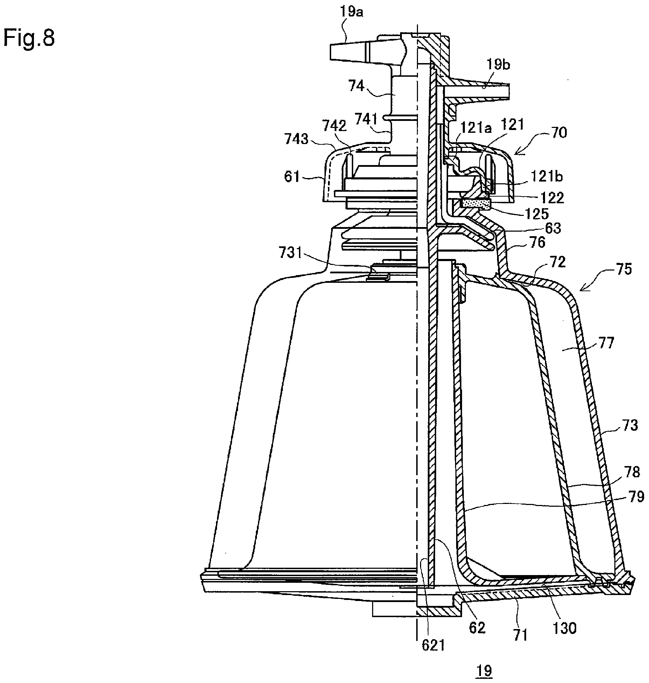

FIG. 8 is a half longitudinal cross-sectional view of the centrifugal bowl 19 of the embodiment. For illustration, a cross section of the centrifugal bowl 19 is illustrated on the right side of the center line, and appearance of the centrifugal bowl 19 is illustrated on the left side of the center line. The centrifugal bowl 19 includes a stator 70 and a rotor 75, which is rotatable with respect to the stator 70. The stator 70 includes a cylindrical bowl head 74 made of polycarbonate and a bowl-shaped cover 61 formed to be connected to one end of the bowl head 74. The bowl head 74 is provided with the inlet 19a and the outlet 19b. To the bowl head 74, an inlet pipe 62 extending to the rotor 75 side is connected. The inlet pipe 62 is connected to the inlet 19a to form an in-pipe flow path 621.

At a part that is outside the inlet pipe 62 and inside the cover 61, a ring cap 121 made of silicone rubber is provided. It is preferable that the ring cap 121 be appropriately elastic and be made of a material having corrosion resistance. The ring cap 121 is formed in a cap shape, a small diameter side 121a is fixed to the bowl head 74, and a large diameter side 121b is secured to the outer diameter of a resin ring 122 such that the large diameter side 121b is pressed by the resin ring 122 and a rib 742. The rib 742 is provided toward the inner circumferential direction on the inner surface of the cover 61 intermittently. A shoulder portion 743 connected to apart where the cover 61 and a neck portion 741 are connected is formed in a gently curved shape. The resin ring 122 is a ring made of a hard resin such as phenol resin and is formed substantially annularly. The resin ring 122 is arranged to abut on a fixing ring 125 to be described later and is held on the stator 70 side.

The rotor 75 includes a side wall 73 made of polycarbonate and formed in a bell shape, a shoulder portion 76 having a reduced diameter at one end of the fixing ring 125, and a bottom plate 71 that is fitted to close the other end of the fixing ring 125. Inside the fixing ring 125, the rotor 75 includes an outer shell 78 and an inner shell 79. The outer shell 78 is in a bell shape and is provided with a support part 731 on its end intermittently. The rotor 75 is structured such that the support part 731 abuts on the inner periphery side of the shoulder portion 76. The side wall 73 is in a funnel shape and structured to fit with the outer shell 78 on both ends thereof. On an end of the shoulder portion 76, the fixing ring 125 made of ceramic, which has high sliding durability, such as alumina is fitted, and the fixing ring 125 is structured to abut on the above-described resin ring 122. Between the lower surface of the inner shell 79 and the upper surface of the bottom plate 71, a substantially discoid bottom flow path 130 is formed.

The bottom plate 71 is a substantially discoid member formed with a depressed part in the central portion of the bottom plate 71. The depressed part is formed to receive blood that has flown down from the in-pipe flow path 621. In addition, between the lower surface of the inner shell 79 and the upper surface of the bottom plate 71, a bottom flow path 130 expanding in substantially discoid shape is formed. The bottom flow path 130 formed between the inner shell 79 and the bottom plate 71 communicates with the in-pipe flow path 621 of the inlet pipe 62. The bottom flow path 130 also communicates with a blood storage space 77 formed by the outer peripheral of the outer shell 78 and the inner peripheral of the side wall 73. The blood storage space 77 has a tapered shape having an inner diameter gradually reduced toward the outlet 19b side. Volume of the blood storage space 77 is preferably about 50 to 1000 ml and more preferably about 100 to 300 ml.

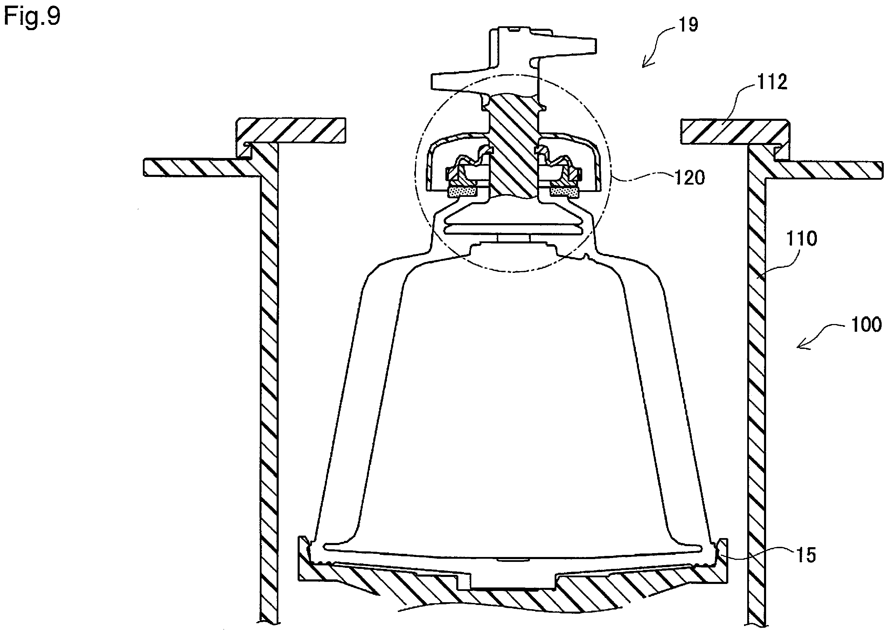

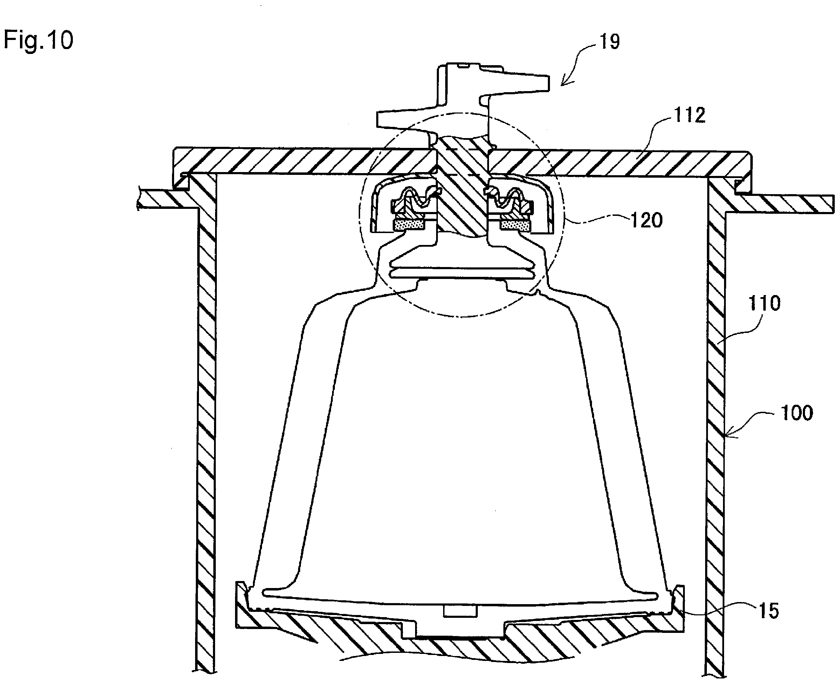

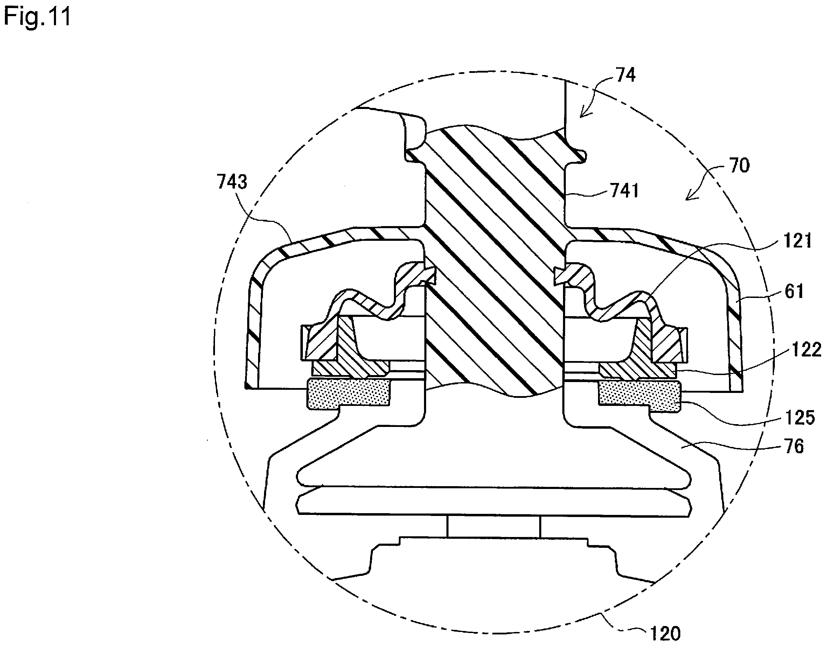

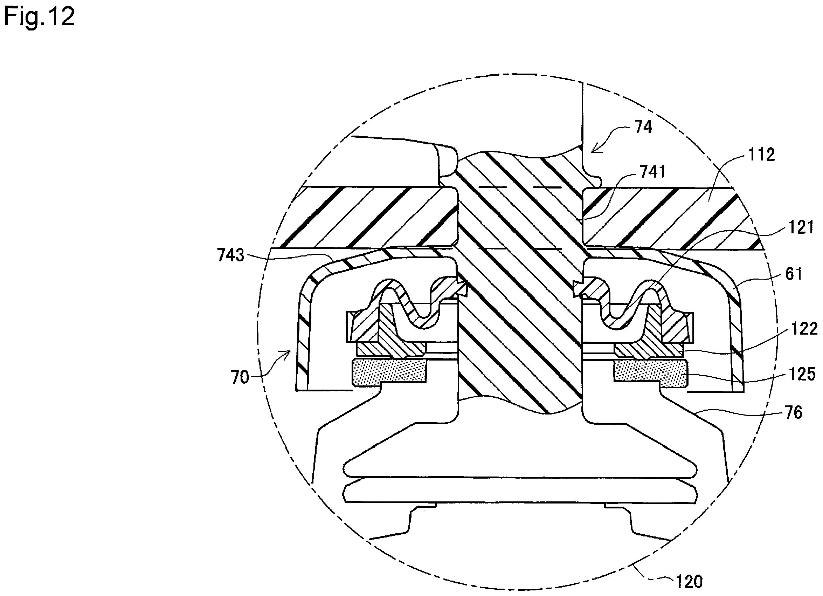

FIG. 9 is a side view of the blood component separation device 100 in a state where the centrifugal bowl 19 is arranged therein but not yet set. FIG. 10 is a side view of the blood component separation device 100 in a state where the centrifugal bowl 19 is arranged therein and has been set. FIG. 11 is an enlarged view of a seal mechanism portion 120. FIG. 11 is an enlarged view of FIG. 9. FIG. 12 is an enlarged view of the seal mechanism portion 120. FIG. 12 is an enlarged view of FIG. 10. The centrifugal bowl 19 is arranged inside a centrifugal tank 110 included in the blood component separation device 100. The centrifugal tank 110 is provided with a lid portion 112 and the centrifugal bowl driving device 15. The centrifugal tank 110 and the lid portion 112 are both made of transparent members. The lid portion 112 is structured openable/closable as illustrated in FIGS. 9 and 10. When the lid portion 112 is closed, the lid portion 112 abuts on the shoulder portion 743 of the centrifugal bowl 19 and the lid portion 112 abuts on the neck portion 741 while pushing down the centrifugal bowl 19. The centrifugal bowl driving device 15 is a part where the bottom plate 71 of the centrifugal bowl 19 abuts when the centrifugal bowl 19 is inserted in the centrifugal tank 110.

The seal mechanism portion 120 has a function of sealing between the stator 70 and the rotor 75 when they abut. On the stator 70 side, the ring cap 121 and the resin ring 122 are provided and on the rotor 75 side, the fixing ring 125 is provided. When the resin ring 122 and the fixing ring 125 abut, the sealing function is exerted. The centrifugal bowl 19 arranged in the centrifugal tank 110 is pressed against the centrifugal bowl driving device 15 side due to the shape of the shoulder portion 743 abutting on the lid portion 112 when the lid portion 112 is closed. As a result, the bottom plate 71 is, for example, stuck to the centrifugal bowl driving device 15. Since the centrifugal bowl driving device 15 is coupled to a rotatably driving device, the centrifugal bowl driving device 15 can rotate the rotor 75 abutting thereon.

The centrifugal bowl 19 and the blood component separation device 100 configured as described above can centrifuge whole blood collected from the blood donor and collect platelet PLT. Strictly speaking, high concentration platelet PLT solution containing plasma PPP, which is difficult to separate, in addition to platelet PLT can be collected. Next, a flow of blood inside the centrifugal bowl 19 will be briefly described.

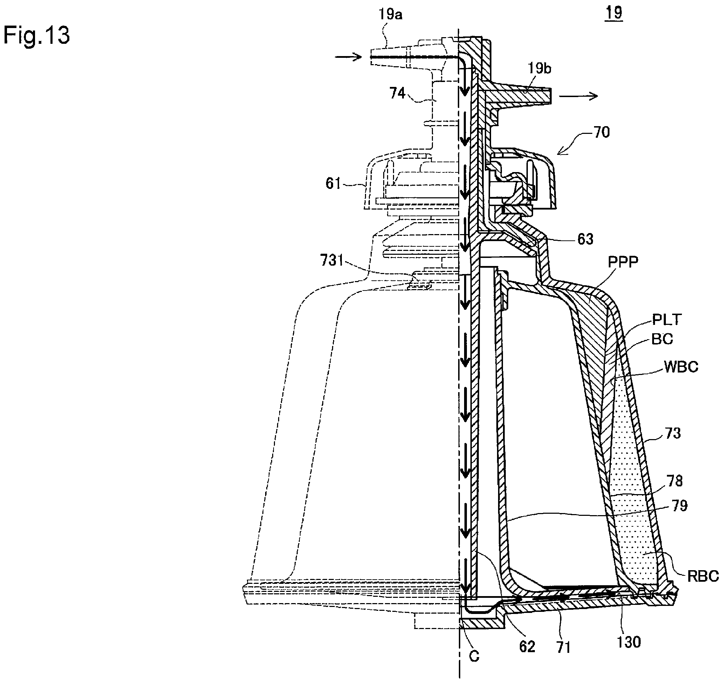

FIG. 13 is a half longitudinal cross-sectional view of a state where whole blood is supplied in the centrifugal bowl 19 and blood components are separated by centrifugal force. For illustration, a cross section of the centrifugal bowl 19 is illustrated on the right side of the center line, and appearance of the centrifugal bowl 19 is illustrated on the left side of the center line with a broken line. Liquid flowing into the centrifugal bowl 19 is carried into the centrifugal bowl 19 through a tube connected to the inlet 19a. The inlet 19a and the in-pipe flow path 621 provided in the inlet pipe 62 are connected, and liquid flowing from the in-pipe flow path 621 is carried to the bottom flow path 130. In the bottom flow path 130, due to the rotor 75 in rotation, blood is carried in the outer circumferential direction of the rotor 75, and liquidmoves to the blood storage space 77. The centrifuged liquid flows from the blood storage space 77, through shoulder portion flow path 72, and through an outflow passage 63. The liquid then moves along the inner wall of the shoulder portion 76 due to centrifugal force of the centrifugal bowl driving device 15 and is carried to the outlet 19b.

When blood flows from the inlet 19a, centrifugal force caused by rotation of the rotor 75 forms a red blood cell RBC layer, a white blood cell WBC layer, a buffy coat BC layer, a platelet PLT layer, and a plasma PPP layer in the blood storage space 77 formed by the outer shell 78 and the side wall 73 in a descending order of specific gravity from the outer side. At this time, since white blood cell WBC and platelet PLT have close specific gravities, it is difficult to separate them. Therefore, a buffy coat BC layer containing white blood cells WBC and platelets PLT is formed. Breakdown of whole blood is typically plasma PPP about 55%, red blood cell RBC about 43.2%, white blood cell WBC about 1.35%, and platelet PLT about 0.45%. In the centrifugal bowl 19, the outflow passage 63 formed a little upper than the middle point of the inlet pipe 62 is formed in the inner peripheral portion. Thus, in the blood storage space 77, whole blood flows from the plasma PPP formed on the inner peripheral, through the outlet 19b, and to the outside of the centrifugal bowl 19.

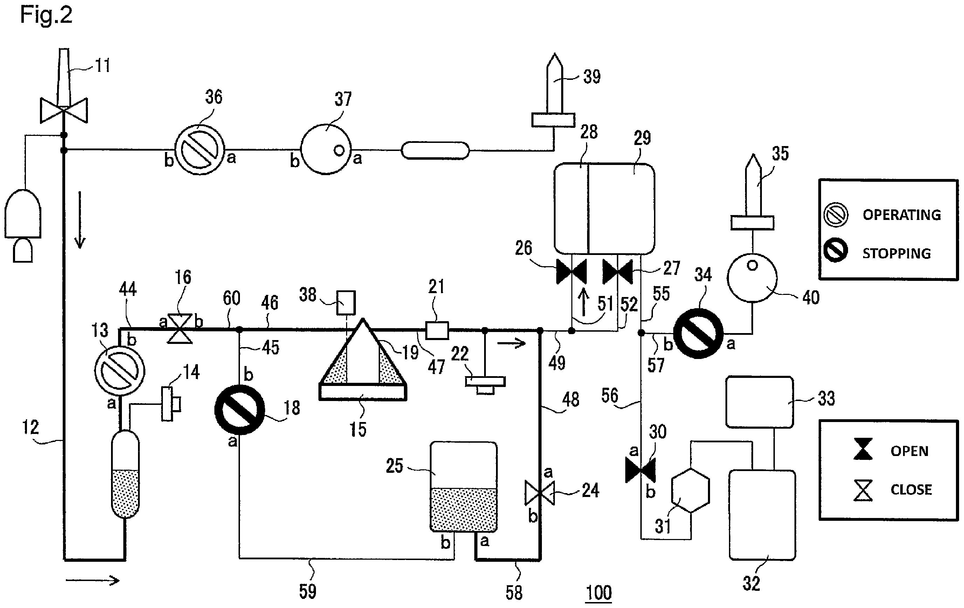

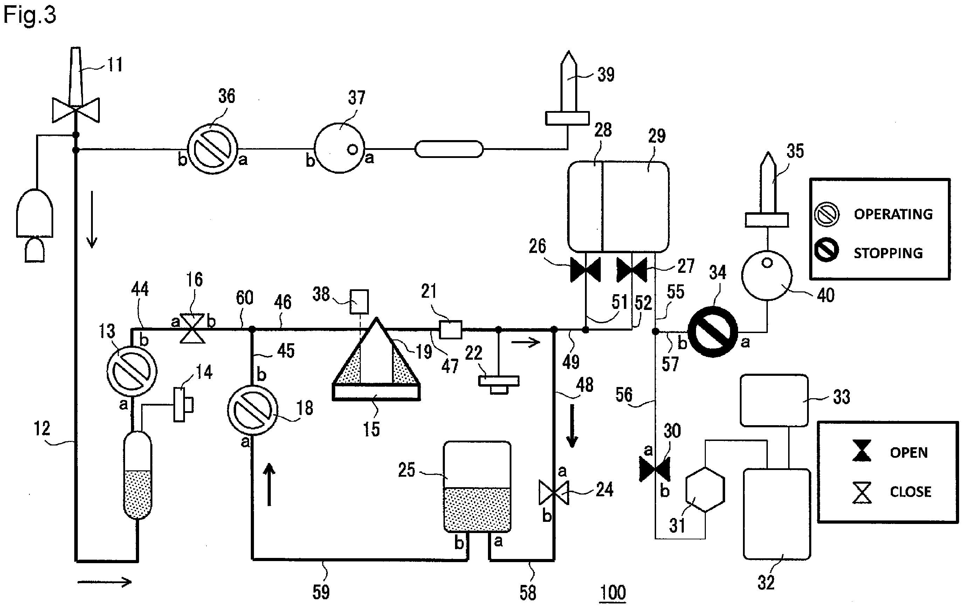

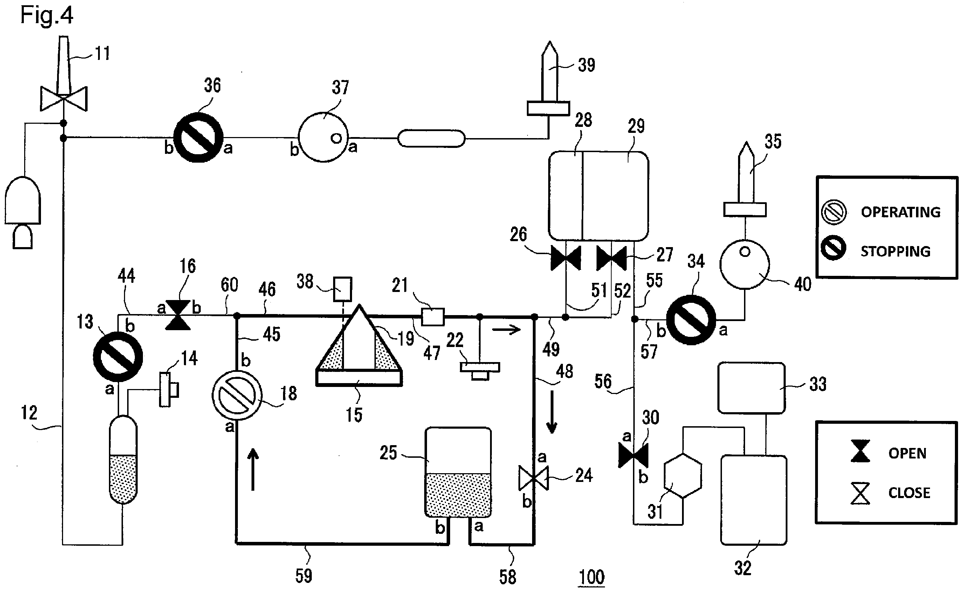

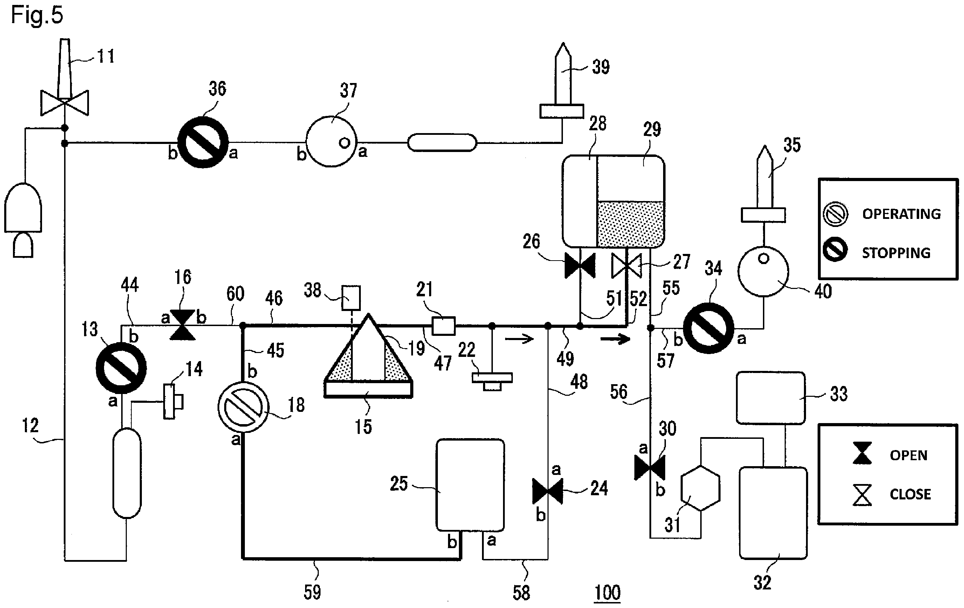

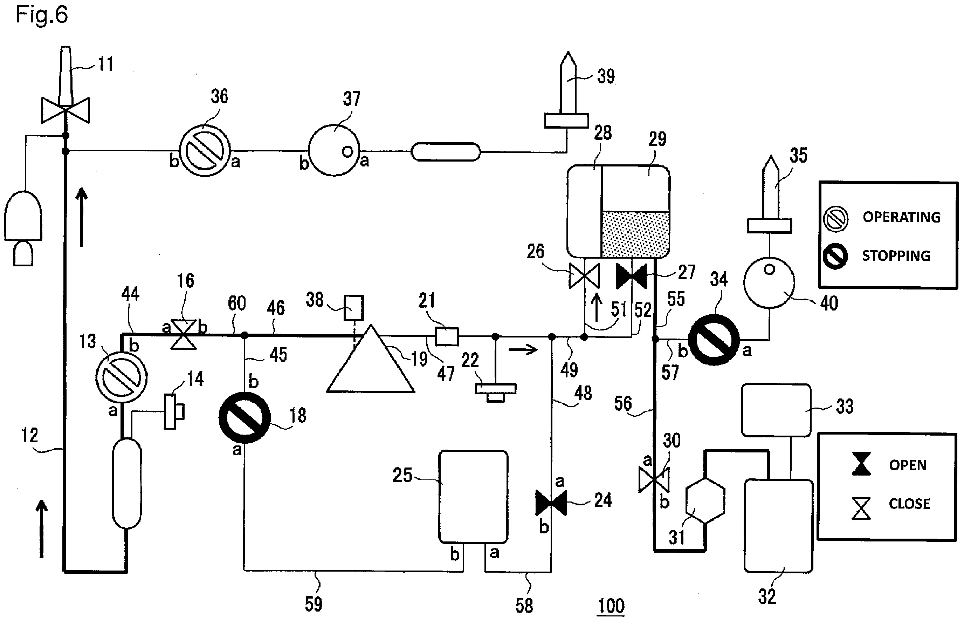

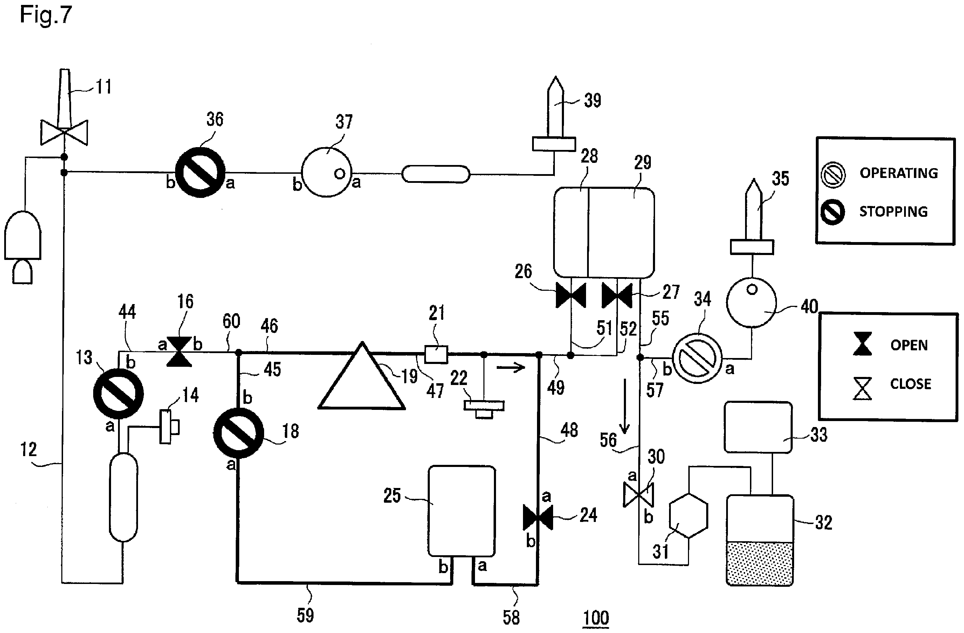

Next, the operation of the blood component separation device configured as described above will be described with reference to FIGS. 1 to 7. FIG. 2 illustrates a second process. FIG. 3 illustrates a third process (critical flowprocess) . FIG. 4 illustrates a fourth process (circulation flow process). FIG. 5 illustrates a process of collecting low concentration platelet PLT solution in a fifth process (acceleration process). FIG. 6 illustrates a process of storing high concentration platelet PLT solution in the fifth process (acceleration process). FIG. 7 illustrates a process of collecting low concentration platelet PLT solution in the fifth process (acceleration process). The blood component separation device 100 is directed to collect high concentration platelet PLT solution. The pumps (the second blood pump 18, the third blood pump 34, and the ACD pump 36) illustrated in FIGS. 1 to 7, indicate that they are in operation when they are white and indicate that they stop when they are black. Similarly, valves (the first on-off valve 16, the second on-off valve 24, the third on-off valve 26, the fourth on-off valve 27, and the fifth on-off valve 30) indicate that they are open when they are white and indicate that they are closed when they are black.

First, The ACD pump 36 and the first blood pump 13 are driven to supply ACD solution for preventing coagulation of blood to the centrifugal bowl 19 through the first on-off valve 16, which is open, and a priming process (first process) of the centrifugal bowl 19 is performed. Priming is a process of applying ACD solution on parts inside the donor tube 12, a first pump, and the centrifugal bowl 19 where blood comes in contact so as to prevent coagulation when blood flows therethrough. ACD solution is supplied from the ACD storage bottle, which is not illustrated and to which the ACD bottle needle 39 is connected. From the priming process, the centrifugal bowl 19 is rotated at a predetermined speed of rotation. When the priming process is completed, the collection needle 11 is punctured into the blood donor as illustrated in FIG. 1 and whole blood collection is started. At this time, the ACD pump 36 is also driven to supply ACD solution to the donor tube 12 and mix the ACD solution into the whole blood. The whole blood is supplied into the centrifugal bowl 19. Here, the ACD solution is supplied such that the ACD solution is about 20% of a mixture of whole blood and the ACD solution.

When whole blood is supplied to the centrifugal bowl 19 in rotation, air inside the centrifugal bowl 19 is pushed by plasma PPP and the air flows out through an outflow portion positioned on the inner peripheral of the centrifugal bowl 19 as illustrated in FIG. 1 (with a dotted line). The air flowing out flows through the third on-off valve 26, which is open, and stored in the air bag 28. In the centrifugal bowl 19, the supplied whole blood is applied with centrifugal force, separating whole blood into components.

Next, when the turbidity sensor 21 detects change of fluids flowing in the tube 47 from air to plasma PPP, the third on-off valve 26 is closed as illustrated in FIG. 2 and the second on-off valve 24 is opened to store plasma PPP overflowing from the centrifugal bowl 19 in the plasma bag 25. The second process has been described above. At first, only plasma PPP flows out from the centrifugal bowl 19.

Next, when some amount of plasma PPP (30 ml in the embodiment) is stored in the plasma bag 25, the second blood pump 18 is driven as illustrated in FIG. 3 to collect whole blood from a blood donor, and the plasma PPP stored in the plasma bag 25 is mixed into the whole blood, and the mixture is supplied to the centrifugal bowl 19. The third process (critical flow process) has been described above.

Next, when the interface sensor 38 detects that the interface between buffy coat BC and platelet PLT comes at a predetermined position, the first on-off valve 16 is closed as illustrated in FIG. 4, and the circulation flow process (fourth process) is performed while the second blood pump 18 is kept driven. The circulation flow process is for circulating the plasma PPP in the plasma bag 25 through the second blood pump 18, the centrifugal bowl 19, and the second on-off valve 24 and back to the plasma bag 25. In the circulation flow process of the embodiment, plasma PPP is circulated through the centrifugal bowl 19 for about 30 to 40 seconds at a rate of about 100 ml/min. Through this process, particulate material concentration in buffy coat BC is reduced. Thus, white blood cells WBC having a larger specific gravity comparing to platelets PLT settle on the outer side of buffy coat BC. More specifically, platelets PLT and white blood cells WBC can be separated more clearly.

Next, after the circulation flow process for a certain time, the acceleration process (fifth process) illustrated in FIG. 5 is started. In the acceleration process, the rotational frequency of the second blood pump 18 is controlled, thereby gradually increasing the rotational frequency to sequentially increase the flow rate of plasma PPP. In the embodiment, the flow rate of plasma PPP is accelerated by 10 ml/min in each several seconds starting from 100 ml/min to 200 ml/min in 20 to 30 seconds. The acceleration applies upward force to platelet PLT, whereby platelet PLT is discharged outside the centrifugal bowl 19 through the outflow passage 63. The acceleration does not discharge white blood cells WBC and red blood cells RBC having a large specific gravity through the outflow passage 63 because centrifugal force is larger than upward force.

The concentration of discharged platelets PLT, white blood cells WBC, andredbloodcells RBC changes as follows. First, platelets PLT are discharged, and discharged amount of platelets PLT tends to gradually increase, exceed the max rate, and then gradually decrease. Similarly, the discharged amount of white blood cells WBC gradually increases, exceeds the max rate, and then gradually decreases. When the turbidity sensor 21 detects platelet PLT solution, the second on-off valve 24 is closed and the fourth on-off valve 27 is opened as illustrated in FIG. 5. Thus, platelet PLT solution can be stored in the platelet intermediate bag 29.

Next, when the concentration of platelets PLT detected by the turbidity sensor 21 is less than a predetermined amount, the process moves to a retransfusion process illustrated in FIG. 6. The rotation of the centrifugal bowl 19 is stopped and the first on-off valve 16 is opened to rotate the first blood pump 13 in the reverse direction, whereby the retransfusion for returning blood remaining in the centrifugal bowl 19 to the blood donor is started. The first bloodpump 13 is driven at the reverse rotation speed that is twice the normal rotation speed to reduce retransfusion time. The second blood pump 18 is also driven as necessary to retransfuse plasma PPP that is excess in collection and stored in the plasma bag 25.

Upon completion of the retransfusion, rotation of the centrifugal bowl 19 is started and the first blood pump 13 is normally rotated again to restart blood collection. The cycle from FIGS. 1 to 6 is repeated. The cycle is usually performed three times or four times until a predetermined amount of platelet PLT is reserved. When the processes are completed within three cycles, the collection needle 11 is removed from the blood donor upon completion of the retransfusion in the third cycle and the blood collection is completed. As to the rotational frequency of the used centrifugal bowl 19, the centrifugal bowl 19 is rotated at, for example, 500 rpm during priming operation and is rotated at, for example, 4000 to 6000 rpm for separating a predetermined blood component.

Next, the fifth on-off valve 30 is opened to inject platelet PLT solution stored in the platelet intermediate bag 29 into the platelet bag 32 through the leukocyte removing filter 31. At this time, air existing in the platelet bag 32 moves to the air bag 33. After confirming that the whole of platelet PLT solution stored in the platelet intermediate bag 29 is discharged, the third blood pump 34 is driven to inject platelet storage liquid to the platelet bag 32 by the platelet bottle needle 35 connected to a platelet storage liquid bottle through the second sterile filter 40. Thereafter, two tubes of the platelet bag are sealed. Through this process, the platelet bag 32 storing high concentration platelet PLT solution is completed.

Because the centrifugal bowl 19 according to the embodiment is structured as described above, operation and effect to be described below are provided.

First, the centrifugal bowl 19 according to the embodiment can suppress high-pitched abnormal noise generation in the priming process. In the blood component separation device 100 including the centrifugal bowl 19 including the stator 70 provided with the inlet 19a and the outlet 19b of blood or a blood component and the rotor 75 provided rotatably with respect to the stator 70, the centrifugal bowl 19 according to the embodiment seals the rotating and sliding portion between the stator 70 and the rotor 75 by pressing the portion with a predeterminedpressing force. The predeterminedpressing force is 500 grams or less.

More specifically, the structure of the seal mechanism portion 120 is characterized in that the rotor 75 includes the annular fixing ring 125, the stator 70 includes the resin ring 122, which is a resin ring in contact with the fixing ring 125 and sliding thereon, and the ring cap 121 holding the resin ring 122, the ring cap 121 applies a predetermined pressing force to the resin ring 122 when the centrifugal bowl 19 is set in the blood component separation device 100, and the rubber hardness of the ring cap 121 is between 60 and 66 inclusive.

As described in the description of object, abnormal noise is unfortunately generated in priming, performed in the above-described first process, for applying ACD solution. The applicant has investigated to specify a cause of the abnormal noise from various viewpoints including the structure of the centrifugal tank 110, friction of the seal mechanism portion 120, and pressing pressure and vibration adsorption of the centrifugal bowl 19. As a result, the applicant has confirmed that abnormal noise is generated due to a stick-slip phenomenon, which occurs in the seal mechanism portion 120. It has been confirmed that a stick-slip phenomenon occurs in a part where the resin ring 122 and the fixing ring 125 abut. It is considered that the stick-slip phenomenon generates chattering during relative movement of the fixing ring 125 and the resin ring 122, and the chattering appears as vibration or abnormal noise. More specifically, it has been found that duringmovement of the fixing ring 125 with respect to the resin ring 122, the stick-slip phenomenon in which sticking and moving are repeated is generated under certain conditions, and the stick-slip phenomenon causes to generate abnormal noise.

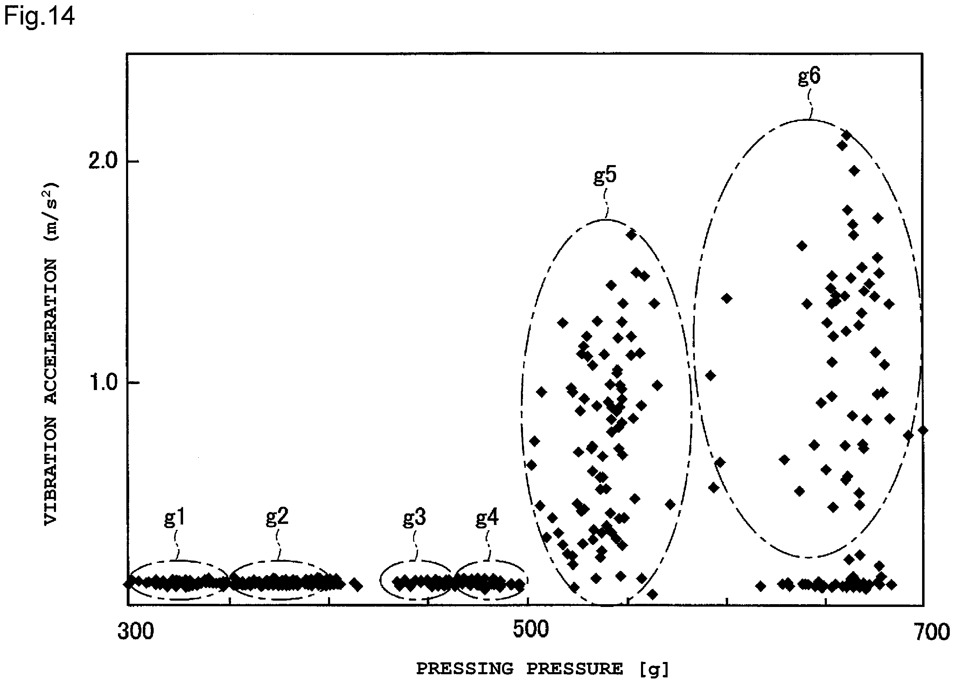

It is considered that the stick-slip phenomenon is affected by friction generated between the fixing ring 125 and the resin ring 122. Therefore, the applicant performed an experiment while changing the hardness of the ring cap 121 supporting the resin ring 122. FIG. 14 illustrates a graph of correlation between pressing pressure and vibration acceleration. The pressing pressure indicates force of pressing the centrifugal bowl 19 to the centrifugal bowl driving device 15 in unit of grams. The vibration acceleration indicates vibration detected by a detector, which is not illustrated and attached to the centrifugal tank 110, in unit of m/s². First to sixth groups g1 to g6 are respective results obtained by changing the hardness of the ring cap 121.

The first group g1 indicating data of vibration acceleration generated between the fixing ring 125 and the resin ring 122 is experiment data when the hardness of the ring cap 121 is set in a rough range from 55 to 59. The second group g2 includes data when the hardness of the ring cap 121 is set to 60, the third group g3 includes data when the hardness is set to 63, the fourth group g4 includes data when the hardness is set to 66, the fifth group g5 includes data when the hardness is set to 68, and the sixth group g6 includes data when the hardness is set to set to 70. The amounts provided as the rubber hardness of the ring cap 121 indicate standard hardness measured by the testing method specified in JIS K6253, which is a result of a test using a durometer. The rubber hardness of the first to sixth groups g1 to g6 is adjusted in the stage of raw material, and the rubber hardness of the ring cap 121 is uniform. In this manner, the rubber hardness of the ring cap 121 and force of pressing the centrifugal bowl 19 were changed and vibration of the centrifugal tank 110 was detected.

As a result of the experiment, vibration causing abnormal noise was not detected under conditions where the pressing pressure is 500 grams or less as illustrated in FIG. 14. In addition, generation of abnormal noise in the audible range was not confirmed under the same conditions. Furthermore, generation of vibration or abnormal noise was not confirmed under conditions where the hardness of the ring cap 121 is 66 or less. In addition, change of the friction coefficient generated between the fixing ring 125 and the resin ring 122 was also studied while changing the surface roughness of the fixing ring 125 and the resin ring 122 and changing mixture of materials of the fixing ring 125. However, remarkable effect could not be provided although a little improvement could be seen. The reason is considered to be that adjusting range is small because the surfaces of the fixing ring 125 and the resin ring 122 used for the centrifugal bowl 19 are originally smooth and the friction coefficients is small.

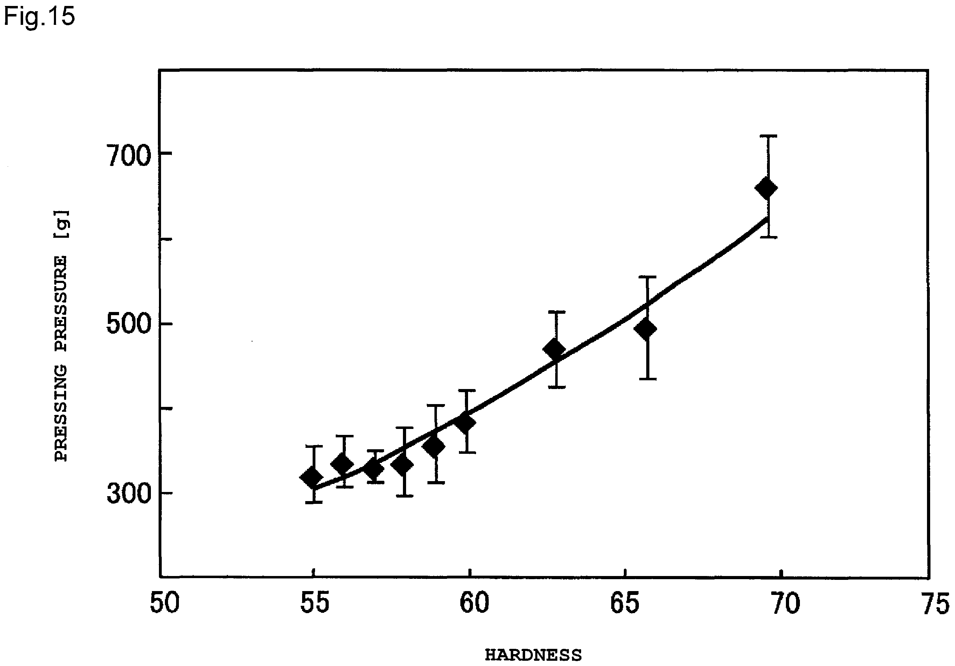

FIG. 15 illustrates a graph of correlation between the hardness and the pressing pressure of the ring cap 121. From FIG. 15, it can be seen that the pressing pressure of the centrifugal bowl 19 linearly increases by increasing the hardness of the ring cap 121. Therefore, the pressing pressure to the centrifugal bowl 19 can be adjusted by selecting the hardness of the ring cap 121. From the result of FIG. 14, the pressing pressure of 500 grams or less can be realized by using the ring cap 121 that provided data in the first to fourth groups g1 to g4, that is, the ring cap 121 having a rubber hardness set in a range of 55 to 66 in the centrifugal bowl 19.

It is considered that the correlation between the rubber hardness of the ring cap 121 and the pressing pressure of the centrifugal bowl 19 is generated due to the way of holding the centrifugal bowl 19 with respect to the blood component separation device 100. Specifically, upon disposing the centrifugal bowl 19 in the blood component separation device 100, the centrifugal bowl 19 is f ixedby suction at a predetermined position of the centrifugal bowl driving device 15, which is arranged inside the centrifugal tank 110, and the lid portion 112 is closed. The relation between the centrifugal bowl 19 and the lid portion 112 is described above. That is, the size relationship of the centrifugal bowl 19 and the lid portion 112 is set such that the lid portion 112 interferes with the shoulder portion 743 of the centrifugal bowl 19 in a state where the centrifugal bowl 19 is arranged in the centrifugal bowl driving device 15. Therefore, closing of the lid portion 112 pushes the centrifugal bowl 19 down toward the centrifugal bowl driving device 15 along the taper of the shoulder portion 743 by a few millimeters as a result. The distance by which the centrifugal bowl 19 is pushed down is absorbed by the seal mechanism portion 120. More specifically, the distance is absorbed by deformation of the ring cap 121 as illustrated in FIGS. 11 and 12. Since the deformation of the ring cap 121 varies depending on the rubber hardness of the ring cap 121, the relationship between the pressing pressure and the rubber hardness illustrated in FIG. 15 is provided. In other words, the pressing pressure therefore changes depending on the rubber hardness of the ring cap 121.

As described above, the pressing pressure of the ring cap 121 could be changed by adjusting the rubber hardness of the ring cap 121, and generation of the above-described abnormal noise due to the stick-slip phenomenon could be prevented by setting the rubber hardness to about 66 or less. It is considered that the stick-slip phenomenon causes repeated moving and sticking of the resin ring 122 with respect to the fixing ring 125 when the fixing ring 125 rotates and moves with respect to the resin ring 122. It is then considered that the moving and sticking caused generation of high-pitched abnormal noise depending on the relationship with the speed of rotation of the centrifugal bowl driving device 15. Therefore, the friction force generated between the resin ring 122 the fixing ring 125 could be changed and thus generation of abnormal noise could be prevented by varying the rubber hardness of the ring cap 121. However, in relation to an issue of sealing performance of the seal mechanism portion 120, appropriate sealing performance may not be provided with the ring cap 121 having too low rubber hardness. Water leak was checked in a test of rotating the centrifugal bowl 19 containing artificial blood and surfactant, and water leak was not found in any of the first to sixth groups g1 to g6. However, in consideration anticipating the safety factor, it is considered that the preferable rubber hardness of the ring cap 121 is 60 or more.

From the consideration, the appropriate hardness of the ring cap 121 is roughly between 60 and 66 inclusive. When the ring cap 121 is structured as described above, the pressing pressure of the centrifugal bowl 19 can be 500 grams or less and abnormal noise generated from the centrifugal bowl 19 can be suppressed. High-pitched abnormal noise generated from the centrifugal bowl 19 is confusing with mechanical noise and may impart a sense of anxiety to a blood donor lying right next to the blood component separation device 100 or an operator who collects blood. However, through the use of the centrifugal bowl 19 according to the embodiment, such anxiety can be resolved.

The specific embodiment of the present invention has been described in detail, but the present invention is not limited to the above-described embodiment and various applications are possible.

For example, change of the centrifugal bowl 19 is not prevented as long as the essential structure of the seal mechanism portion 120 is the same. In addition, change of the structure of the blood component separation device 100 is not prevented. Further, change of the material of the centrifugal bowl 19, which has been exemplified, is not prevented without departing from the scope of the invention, which is defined by the claims.

### Reference Signs List

- 61: cover
- 62: inlet pipe
- 63: outflow passage
- 70: stator
- 71: bottom plate
- 72: shoulder portion flow path
- 73: side wall
- 74: bowl head
- 75: rotor
- 76: shoulder portion
- 77: blood storage space
- 78: outer shell
- 79: inner shell
- 100: blood component separation device
- 110: centrifugal tank
- 112: lid portion
- 120: seal mechanism portion
- 121: ring cap
- 122: resin ring
- 125: fixing ring
- 130: bottom flow path
- 621: in-pipe flow path
- 731: support part
- 741: neck portion
- 742: rib
- 743: shoulder portion
- BC: buffy coat
- PLT: platelet
- PPP: plasma
- RBC: red blood cell
- WBC: white blood cell

## Claims

1. A blood component separation device (100) comprising a centrifugal separator including a stator (70) provided with an inlet and an outlet of blood or a blood component and a rotor (75) provided rotatably with respect to the stator (70),
**characterized in that**
a rotating and sliding portion between the stator (70) and the rotor (75) is sealed by pressing with predetermined pressing force, the predetermined pressing force is 500 grams or less,
the rotor (75) includes an annular fixing ring (125),
the stator (70) includes a resin ring (122) and a ring cap (121) that holds the resin ring (122), the resin ring (122) being in contact with the fixing ring (125) and sliding thereon,
the ring cap (121) applies the predetermined pressing force to the resin ring (122) when the centrifugal separator is set in the blood component separation device (100), and
a rubber hardness of the ring cap (121) is between 60 and 66 inclusive when measured by the testing method specified in JIS K 6253.

2. A centrifugal separator that comprises a stator (70) provided with an inlet and an outlet of blood or blood component and a rotor (75) provided rotatably with respect to the stator (70), **characterized in that** a rotating and sliding portion between the stator (70) and the rotor (75) is sealed by pressing with predetermined pressing force, the predetermined pressing force is 500 grams or less, and that is used in a state where the centrifugal separator is set in a blood component separation device (100), wherein
the rotor (75) includes an annular fixing ring (125),
the stator (70) includes a resin ring (122) and a ring cap (121) that holds the resin ring (122), the resin ring (122) being in contact with the fixing ring (125) and sliding thereon,
the ring cap (121) applies predetermined pressing force to the resin ring (122) when the centrifugal separator is set in the blood component separation device (100), and
a rubber hardness of the ring cap (121) is between 60 and 66 inclusive when measured by the testing method specified in JIS K 6253, so as to set the predetermined pressing force to 500 grams or less.

## Patentansprüche

1. Blutkomponententrennvorrichtung (100), welche einen Zentrifugalabscheider umfasst, welcher einen mit einem Einlass und einem Auslass für Blut oder eine Blutkomponente ausgestatteten Stator (70) und einen im Hinblick auf den Stator (70) drehbaren Rotor (75) aufweist,
**dadurch gekennzeichnet, dass**
ein Dreh- und Gleitbereich zwischen dem Stator (70) und dem Rotor (75) durch Drücken mit einer vorbestimmten Druckkraft von 500 Gramm oder weniger abgedichtet wird,
der Rotor (75) einen Befestigungsring (125) aufweist,
der Stator (70) einen mit dem Befestigungsring (125) in Berührung stehenden und auf diesem gleitenden Kunstharzring (122) und eine den Kunstharzring (122) haltende Ringkappe (121) aufweist,
die Ringkappe (121) die vorbestimmte Druckkraft auf den Kunstharzring (122) ausübt, wenn der Zentrifugalabscheider in der Blutkomponententrennvorrichtung (100) platziert ist, und eine Granulathärte der Ringkappe (121), wenn nach dem Prüfverfahren gemäß JIS K 6253 gemessen, zwischen jeweils einschließlich 60 und 66 ist.

2. Zentrifugalabscheider, welcher einen mit einem Einlass und einem Auslass für Blut oder eine Blutkomponente ausgestatteten Stator (70) und einen im Hinblick auf den Stator (70) drehbaren Rotor (75) umfasst,
**dadurch gekennzeichnet, dass**
ein Dreh- und Gleitbereich zwischen dem Stator (70) und dem Rotor (75) durch Drücken mit einer vorbestimmten Druckkraft von 500 Gramm oder weniger abgedichtet wird, und dieser in einem Zustand benutzt wird, wo der Zentrifugalabscheider in der Blutkomponententrennvorrichtung (100) platziert ist, wobei
der Rotor (75) einen Befestigungsring (125) aufweist,
der Stator (70) einen mit dem Befestigungsring (125) in Berührung stehenden und auf diesem gleitenden Kunstharzring (122) und eine den Kunstharzring (122) haltende Ringkappe (121) aufweist,
die Ringkappe (121) die vorbestimmte Druckkraft auf den Kunstharzring (122) ausübt, wenn der Zentrifugalabscheider in der Blutkomponententrennvorrichtung (100) platziert ist, und
eine Granulathärte der Ringkappe (121), wenn nach dem Prüfverfahren gemäß JIS K 6253 gemessen, zwischen jeweils einschließlich 60 und 66 ist, so dass die vorbestimmte Druckkraft auf 500 Gramm oder weniger eingestellt wird.

## Revendications

1. Dispositif de séparation de composants sanguins (100) comportant un séparateur centrifuge comprenant un stator (70) muni d'un orifice d'entrée et d'un orifice de sortie de sang ou d'un composant sanguin et un rotor (75) placé en rotation par rapport au stator (70),
**caractérisé en ce que**
une partie tournante et coulissante entre le stator (70) et le rotor (75) est scellée par une pression exercée avec une force de pression prédéterminée, la force de pression prédéterminée est de 500 grammes ou moins,
le rotor (75) comporte une bague de fixation annulaire (125),
le stator (70) comporte une bague en résine (122) et un couvercle annulaire (121) qui maintient la bague en résine (122), la bague en résine (122) étant en contact avec la bague de fixation (125) et coulissant sur celle-ci,
le couvercle annulaire (121) applique la force de pression prédéterminée sur la bague en résine (122) lorsque le séparateur centrifuge est placé dans le dispositif de séparation de composants sanguins (100), et une dureté de caoutchouc du couvercle annulaire (121) est de 60 à 66 compris, mesurée par la méthode d'essai spécifiée dans la norme JIS K 6253.

2. Séparateur centrifuge qui comprend un stator (70) muni d'un orifice d'entrée et d'un orifice de sortie de sang ou d'un composant sanguin et un rotor (75) placé en rotation par rapport au stator (70),
**caractérisé en ce que**
une partie tournante et coulissante entre le stator (70) et le rotor (75) est scellée par une pression exercée avec une force de pression prédéterminée, la force de pression prédéterminée est de 500 grammes ou moins, et est utilisée dans un état où le séparateur centrifuge est placé dans le dispositif de séparation de composants sanguins (100), dans lequel
le rotor (75) comporte une bague de fixation annulaire (125),
le stator (70) comporte une bague en résine (122) et un couvercle annulaire (121) qui maintient la bague en résine (122), la bague en résine (122) étant en contact avec la bague de fixation (125) et coulissant sur celle-ci,
le couvercle annulaire (121) applique la force de pression prédéterminée sur la bague en résine (122) lorsque le séparateur centrifuge est placé dans le dispositif de séparation de composants sanguins (100), et une dureté de caoutchouc du couvercle annulaire (121) est de 60 à 66 compris, mesurée par la méthode d'essai spécifiée dans la norme JIS K 6253, de manière à fixer la force de pression prédéterminée à 500 grammes ou moins.
